(19) Europäisches Patentamt
European Patent Office
Office européen des brevets

(11) EP 0 735 027 B1

(12) **EUROPÄISCHE PATENTSCHRIFT**

(45) Veröffentlichungstag und Bekanntmachung des Hinweises auf die Patenterteilung:
**17.09.1997 Patentblatt 1997/38**

(51) Int. Cl.$^6$: **C07D 229/00**, C08G 18/79

(21) Anmeldenummer: **96101010.5**

(22) Anmeldetag: **25.01.1996**

# (54) Verfahren zur Herstellung von (cyclo)aliphatischen Uretdionen mit verbesserter Farbqualität

Process for the preparation of (cyclo)aliphatic uretdiones with improved color quality

Procédé de préparation d'uretdiones (cyclo)aliphatiques ayant une meilleure qualité de couleur

(84) Benannte Vertragsstaaten:
**AT BE CH DE ES FR GB IT LI NL SE**

(30) Priorität: **25.03.1995 DE 19510956**

(43) Veröffentlichungstag der Anmeldung:
**02.10.1996 Patentblatt 1996/40**

(73) Patentinhaber: **HÜLS AKTIENGESELLSCHAFT**
**45764 Marl (DE)**

(72) Erfinder:
- **Schmitt, Felix, Dr.**
  **D-45701 Herten (DE)**
- **Elmar, Wolf, Dr.**
  **D-45661 Recklinghausen (DE)**

(56) Entgegenhaltungen:
**EP-A- 0 045 995**
**EP-A- 0 173 252**
**EP-A- 0 317 744**
**EP-A- 0 478 990**
**WO-A-93/19049**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 735 027 B1

Printed by Rank Xerox (UK) Business Services
2.14.14/3.4

**Beschreibung**

Gegenstand der vorliegenden Erfindung ist ein Verfahren zur Herstellung von (cyclo)aliphatischen Uretdionen mit verbesserter Farbqualität. Derartige Uretdione, deren Herstellung in der DE-OS 37 39 549 beschrieben sind, lassen sich zu lichtechten PUR-Pulvern verarbeiten. Ein Nachteil dieser (cyclo)aliphatischen Uretdione der DE-OS 37 39 549 ist ihre mehr oder minder starke Verfärbung. Es ist grundsätzlich bekannt, Uretdione durch Dimerisierung von Isocyanaten in Gegenwart bestimmter Katalysatoren herzustellen. So beschreibt die EP-A-173 252 die Verwendung von tert. Phosphinen oder peralkylierten Phosphorigsäuretriamiden als Katalysatoren. Bei der Dimerisierung von (cyclo)aliphatischen Isocyanaten mit tert. Phosphinen werden neben dem erwünschten Uretdion aber auch Isocyanurate erhalten, deren Anwesenheit für eine Reihe von Anwendungen unerwünscht ist, da Isocyanurate trifunktionell sind und zur Vernetzung neigen. Mit diesem Nachteil sind die peralkylierten Phosphorigsäuretriamide nicht behaftet. Diese Katalysatoren neigen aber in Gegenwart von Luftsauerstoff zur Bildung von Phosphorsäuretriamiden, die im Verdacht stehen, cancerogen zu sein. Die WO-A-93/19049 demonstriert, daß N,N-Dialkylaminopyridine an Polymere fixiert werden können, ohne ihre katalytische Aktivität zu verlieren. Nachteil der auf Basis von N,N-Dialkylaminopyridinen hergestellten (cyclo)aliphatischen Uretdione ist ihre mehr oder minder starke Verfärbung. Überraschenderweise konnten diese Verfärbungen weitgehend beseitigt werden, wenn bei der Dimerisierung des Diisocyanates eine dreibindige Phosphorverbindung zugegen war.

Gegenstand der Erfindung ist daher ein Verfahren zur Herstellung von (cyclo)aliphatischen Uretdionen mit verbesserter Farbqualität durch Umsetzung von (cyclo)aliphatischen Diisocyanaten, gegebenenfalls in einem inerten Lösungsmittel, mit Hilfe von 0,5 bis 3 Gew.-% Katalysatoren der allgemeinen Formel I

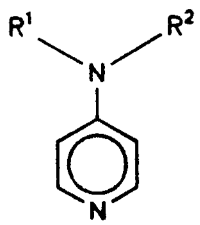

I,

wobei $R^1$ und $R^2$ gleiche oder verschiedene Alkylreste mit 1 bis 8 C-Atomen darstellen oder mit dem am Ring gebundenen N-Atom einen gemeinsamen 5- oder 6-Ring bilden können, der anstelle einer $CH_2$-Gruppe eine $CH$-$CH_3$-Gruppe, eine $N$-$CH_3$-Gruppe oder ein O-Atom enthalten kann, in Gegenwart von 0,1 bis 4 Gew.-% Phosphorverbindungen der allgemeinen Formel II

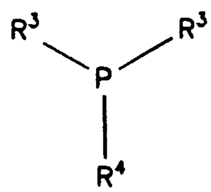

II,

wobei $R^3$ und $R^4$ gleiche oder verschiedene aromatische oder araliphatische Kohlenwasserstoffreste mit 1 bis 10 Kohlenstoffatomen oder $OR^1$ oder OH bedeuten, wobei bei Temperaturen von 0 bis 60 °C zunächst bis zu einem Umsatz von 5 bis 70 % dimerisiert wird und danach das gebildete 1,3-Diazacyclobutandion-2,4 ohne vorhergehende Desaktivierung des Katalysators aus dem Reaktionsgemisch durch Dünnschichtdestillation als Rückstand sowie Katalysator, Phosphorverbindungen und nicht umgesetztes Diisocyanat als Destillat isoliert werden.

Die Katalysatoren werden in Mengen von 0,5 bis 3 Gew.-%, vorzugsweise 1 bis 2 Gew.-%, eingesetzt. Es handelt sich hierbei um N,N-disubstituierte 4-Aminopyridin-Derivate, wie z. B. 4-Dimethylaminopyridin, 4-Diethylaminopyridin, 4-Pyrrolidinopyridin, 4-Piperidinopyridin und 4-(4-Methylpiperidino)-pyridin.

Bei den Phosphorverbindungen handelt es sich um dreibindige Phosphorverbindungen, wie z. B. Triphenylphosphin, Triethylphosphit, Tributylphosphit, Dibutylphosphit und Dibenzylphosphit. Sie werden in Mengen von 0,1 bis 4 Gew.-%, vorzugsweise 0,5 bis 2 Gew.-%, dem zu dimerisierenden Diisocyanat zugesetzt. Die nach dem erfindungsgemäßen Verfahren eingesetzten (cyclo)aliphatischen Diisocyanate enthalten 6 bis 15 C-Atome.

Die Durchführung des erfindungsgemäßen Verfahrens erfolgt derartig, daß zunächst mit Hilfe des beschriebenen Katalysators und der Phosphorverbindungen bis zu einem Umsatz, der die Förderung des Reaktionsgemisches im flüssigen Zustand (bei Raumtemperatur) noch zuläßt (40 bis 60 % Diisocyanat-Umsatz), dimerisiert wird, und danach

das nicht umgesetzte Diisocyanat mit dem Katalysator und der Phosphorverbindung durch Dünnschichtdestillation vom Reaktionsprodukt abgetrennt wird.

Das abdestillierte Diisocyanat (plus Katalysator und Phosphorverbindung) kann wieder zur Reaktion eingesetzt werden.

Die Reaktionstemperatur liegt in einem Bereich von 0 bis 60 °C, vorzugsweise 10 bis 30 °C. Bei höheren Temperaturen macht sich deutlich die katalytische Umwandlung des Uretdions in das entsprechende Isocyanurat bemerkbar.

Die Reaktionszeit, die Zeit, in der z. B. 40 bis 60 % des Diisocyanats umgesetzt sind, hängt (bei konstanter Temperatur) in hohem Maß von der Konzentration sowie von der Art des eingesetzten Katalysators ab. Sie beträgt in der Regel 10 bis 90 h. Die Reaktion kann in polaren Lösungsmitteln, wie Estern, Ethern und Ketonen, oder lösungsmittelfrei durchgeführt werden. Bevorzugt arbeitet man lösungsmittelfrei.

Die Aufarbeitung des Reaktionsgemisches erfolgt, wie bereits angeführt, durch Dünnschichtdestillation bei 100 bis 180 °C und 0,01 bis 0,5 mbar.

Die nach dem erfindungsgemäßen Verfahren hergestellten Uretdione zeichnen sich gegenüber den Uretdionen der DE-OS 37 39 549 durch eine verbesserte Farbqualität aus.

**Beispiele 1 bis 12**

Die in der nachfolgenden Tabelle aufgeführten Uretdione des Isophorondiisocyanats (IPDI) wurden so hergestellt, daß man in einer 1. Stufe IPDI mit dem Katalysator und der Phosphorverbindung bei 25 °C unter $N_2$-Abdeckung bis zu einem Umsatz von 30 bis 40 % umsetzte, und in einem 2. Schritt das nicht umgesetzte IPDI mit dem Katalysator und Phosphorverbindung durch Dünnschichtdestillation bei 140 °C/0,1 mbar vom Reaktionsprodukt, das nur geringe Spuren an Katalysator (0,05 %) und Phosphorverbindung (< 0,1 %) enthielt, abtrennte.

**Tabelle 1:** **Katalytische Dimerisierung von IPDI in Gegenwart von Triphenylphosphin**

| Beispiel | 1 (Vergleich) | 2 (Vergleich) | 3 | 4 | 5 (Vergleich) | 6 (Vergleich) | 7 | 8 |
|---|---|---|---|---|---|---|---|---|
| IPDI | <—— | | | | | | | ——> |
| 4-Dimethylaminopyridin Gew.-% | 1 | 2 | 1 | 2 | - | - | - | - |
| 4-Pyrrolidonpyridin Gew.-% | - | - | - | - | 1 | 2 | 1 | 2 |
| Triphenylphosphin Gew.-% | - | - | 1 | 1 | - | - | 1 | 1 |
| Reaktionszeit [h] | 72 | 25 | 72 | 25 | 41 | 18 | 41 | 18 |
| % NCO | 29,7 | 30,0 | 29,2 | 29,6 | 29,5 | 29,0 | 28,9 | 28,9 |
| $n_D^{25}$ | 1,4932 | 1,4925 | 1,4942 | 1,4940 | 1,4929 | 1,4940 | 1,4948 | 1,4956 |
| Farbe (Hazen) | 267 | 389 | 114 | 112 | 303 | 361 | 130 | 183 |
| ↓ Dünnschichtdestillation 140 °C/0,1 mbar | | | | | | | | |
| % NCO | 17,6 | 17,9 | 17,8 | 17,8 | 17,5 | 17,6 | 17,4 | 17,8 |
| % IPDI | 0,6 | 0,7 | 0,6 | 0,3 | 0,3 | 0,5 | 0,3 | 0,3 |
| Farbzahl Hazen (70 %ig in Butylacetat) | 248 | 227 | 147 | 219 | 308 | 401 | 250 | 223 |

Tabelle 2

| Katalytische Dimerisierung von IPDI in Gegenwart von Tributylphosphit | | | | |
|---|---|---|---|---|
| Beispiel | 9 | 10 | 11 | 12 |
| IPDI | ↔ | | | |
| DMAP Gew.-% | 1 | 2 | - | - |
| 4-Pyrrolidinopyridin Gew.-% | - | - | 1 | 2 |
| Tributylphosphit Gew.-% | 1 | 2 | 1 | 1 |
| Reaktionszeit [h] | 72 | 25 | 41 | 18 |
| % NCO | 29,6 | 29,9 | 29,2 | 29,0 |
| $n_D^{25}$ | 1,4933 | 1,4924 | 1,4942 | 1,4948 |
| Farbzahl (Hazen) | 94 | 97 | 112 | 136 |
| ↓ Dünnschichtdestillation 140 °C/0,1 mbar | | | | |
| % NCO | 17,6 | 17,7 | 17,8 | 17,8 |
| % IPDI | 0,4 | 0,3 | 0,4 | 0,5 |
| Farbzahl (Hazen) (70 %ig in Butylacetat) | 102 | 106 | 118 | 125 |

**Patentansprüche**

1. Verfahren zur Herstellung von (cyclo)aliphatischen Uretdionen mit verbesserter Farbqualität durch Umsetzung von (cyclo)aliphatischen Diisocyanaten, gegebenenfalls in einem inerten Lösungsmittel, mit Hilfe von 0,5 bis 3 Gew.-% Katalysatoren der allgemeinen Formel I

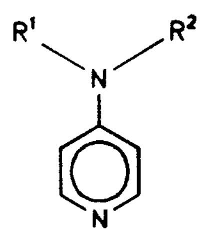

I,

wobei $R^1$ und $R^2$ gleiche oder verschiedene Alkylreste mit 1 bis 8 C-Atomen darstellen oder mit dem am Ring gebundenen N-Atom einen gemeinsamen 5- oder 6-Ring bilden können, der anstelle einer $CH_2$-Gruppe eine $CH-CH_3$-Gruppe, eine $N-CH_3$-Gruppe oder ein O-Atom enthalten kann, in Gegenwart von 0,1 bis 4 Gew.-% Phosphorverbindungen der allgemeinen Formel II

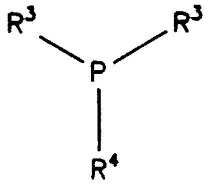

II,

wobei $R^3$ und $R^4$ gleiche oder verschiedene aromatische oder araliphatische Kohlenwasserstoffreste mit 1 bis 10

Kohlenstoffatomen oder $OR^1$ oder OH bedeuten, wobei bei Temperaturen von 0 bis 60 °C zunächst bis zu einem Umsatz von 5 bis 70 % dimerisiert wird und danach das gebildete 1,3-Diazacyclobutandion-2,4 ohne vorhergehende Desaktivierung des Katalysators aus dem Reaktionsgemisch durch Dünnschichtdestillation als Rückstand, sowie Katalysator, Phosphorverbindungen und nicht umgesetztes Diisocyanat als Destillat, isoliert werden.

2. Verfahren nach Anspruch 1,
dadurch gekennzeichnet,
daß die Umsetzung bei Temperaturen von 10 bis 30 °C erfolgt.

3. Verfahren nach mindestens einem der Ansprüche 1 bis 2,
dadurch gekennzeichnet,
daß die Katalysatoren in Mengen von 1 bis 2 Gew.-% eingesetzt werden.

4. Verfahren nach mindestens einem der Ansprüche 1 bis 3,
dadurch gekennzeichnet,
daß die Phosphorverbindungen in Mengen von 0,5 bis 2 Gew.-% eingesetzt werden.

5. Verfahren nach mindestens einem der Ansprüche 1 bis 4,
dadurch gekennzeichnet,
daß nach einem Umsatz von 20 bis 50 % mit der Dünnschichtdestillation begonnen wird.

6. Verfahren nach mindestens einem der Ansprüche 1 bis 5,
dadurch gekennzeichnet,
daß als Katalysator 4-Dimethylaminopyridin, 4-Diethylaminopyridin, 4-Pyrrolidinopyridin, 4-Piperidinopyridin und 4-(4-Methylpiperidino)-pyridin, eingesetzt werden.

7. Verfahren nach mindestens einem der Ansprüche 1 bis 6,
dadurch gekennzeichnet,
daß als Phosphorverbindung Triphenylphosphin, Triethylphosphit, Tributylphosphit, Dibutylphosphit und Dibenzylphosphit, eingesetzt werden.

8. Verfahren nach mindestens einem der Ansprüche 1 bis 7,
dadurch gekennzeichnet,
daß die Umsetzung lösemittelfrei erfolgt.

9. Verfahren nach mindestens einem der Ansprüche 1 bis 8,
dadurch gekennzeichnet,
daß als Diisocyanat Isophorondiisocyanat eingesetzt wird.

## Claims

1. A process for the preparation of (cyclo)aliphatic uretdiones of improved colour quality by reaction of (cyclo)aliphatic diisocyanates, if desired in an inert solvent, with the aid of from 0.5 to 3% by weight of catalysts of the general formula I

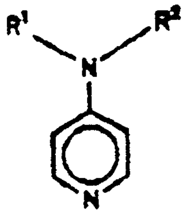

I

in which $R^1$ and $R^2$ are identical or different alkyl radicals of 1 to 8 carbon atoms or, together with the nitrogen atom attached to the ring, can form a conjoint 5-membered or 6-membered ring which can contain, instead of one $CH_2$ group, a $CH\text{-}CH_3$ group, a $N\text{-}CH_3$ group or an oxygen atom, in the presence of from 0.1 to 4% by weight of phosphorus compounds of the general formula II

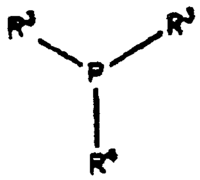

II

in which $R^3$ and $R^4$ are identical or different aromatic or araliphatic hydrocarbon radicals of 1 to 10 carbon atoms or arc $OR^1$ or OH, dimerization first being carried out at temperatures of from 0 to 60°C up to a conversion of from 5 to 70% and then the 1,3-diazacyclobutane-2,4-dione formed being isolated, without prior deactivation of the catalyst, from the reaction mixture by thin-film distillation as the residue, and catalyst, phosphorus compounds and unreacted diisocyanate being isolated from the reaction mixture by thin-film distillation as the distillate.

2. A process according to claim 1, characterized in that the reaction is carried out at temperatures of from 10 to 30°C.

3. A process according to one or both of claims 1 and 2, characterized in that the catalysts are employed in quantities of from 1 to 2% by weight.

4. A process according to at least one of claims 1 to 3, characterized in that the phosphorus compounds are employed in quantities of from 0.5 to 2% by weight.

5. A process according to at least one of clams 1 to 4, characterized in that thin-film distillation is begun after a conversion of from 20 to 50%.

6. A process according to at least one of claims 1 to 5, characterized in that 4-dimethylaminopyridine, 4-diethylaminopyridine. 4-pyrrolidinopyridine, 4-piperidinopyridine and 4-(4-methylpiperidino)-pyridine are employed as catalyst.

7. A process according to at least one of claims 1 to 6, characterized in that triphenylphosphine, triethyl phosphite, tributyl phosphite, dibutyl phosphite and dibenzyl phosphite are employed as phosphorus compound.

8. A process according to at least one of claims 1 to 7, characterized in that the reaction is carried out without solvent.

9. A process according to at least one of claims 1 to 8, characterized in that isophorone diisocyanate is employed as diisocyanate.

## Revendications

1. Procédé pour la fabrication d'uretdiones aliphatiques (cycliques) avec une qualité de couleur améliorée, au moyen de la transformation de diisocyanates aliphatiques (cycliques), le cas échéant dans un solvant inerte, à l'aide de 0,5 à 3 % en poids de catalyseurs selon la formule générale I

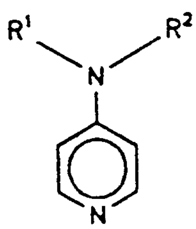

(I)

où $R^1$ et $R^2$ représentent des radicaux alkyles égaux ou différents, comportant 1 à 8 atomes de carbone ou qui peuvent former un cycle à 5 ou 6 atomes en commun avec l'atome d'azote lié au cycle, lequel peut comporter au lieu d'un groupe $CH_2$ un groupe $CH\text{-}CH_3$, un groupe $N\text{-}CH_3$ ou un atome d'oxygène, en présence de 0,1 à 4 % en poids de compose phosphorique selon la formule générale II

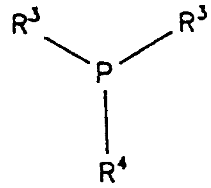

(II)

où $R^3$ et $R^4$ représentent des radicaux hydrocarbures égaux ou différents, aromatiques ou araliphatiques, comportant 1 à 10 atomes de carbone où $OR^1$ ou OH, où l'on dimérise à des températures comprises entre 0°C et 60°C d'abords jusqu'à un taux de transformation de 5 à 70 % et où l'on isole ensuite le 1,3-diazacyclobutanedione-2,4 formé du mélange réactionnel, sans désactivation du catalyseur préalable, comme résidu au moyen d'une distillation à couche mince, et le catalyseur, les composés phosphoriques et le diisocyanate non transformé comme distillat.

**2.** Procédé selon la revendication 1,
caractérisé en ce que
la transformation est effectuée à des températures comprises entre 10°C et 30°C.

**3.** Procédé selon au moins une des revendications 1 à 2,
caractérisé en ce que
les catalyseurs sont mis en oeuvre en quantités de 1 à 2 % en poids.

**4.** Procédé selon au moins une des revendications 1 à 3,
caractérisé en ce que
les composés phosphoriques sont mis en oeuvre en quantités de 0,5 à 2 % en poids.

**5.** Procédé selon au moins une des revendications 1 à 4,
caractérisé en ce que
l'on commence la distillation à couche mince après avoir atteint un taux de transformation de 20 à 50 %.

**6.** Procédé selon au moins une des revendications 1 à 5,
caractérisé en ce que
que l'on utilise comme catalyseur la 4-diméthylaminopyridine, la 4-diéthylaminopyridine, la 4-pyrrolidinopyridine, la 4-pipéridinopyridine et la 4-(4-méthyl-pipéridino)-pyridine.

**7.** Procédé selon au moins une des revendications 1 à 6,
caractérisé en ce que
l'on utilise comme composé phosphorique la triphénylphosphine, le triéthylphosphite, le tributylphosphite, le dibutylphosphite et le dibenzylphosphite.

**8.** Procédé selon au moins une des revendications 1 à 7,
caractérisé en ce que
la transformation a lieu sans solvants.

**9.** Procédé selon au moins une des revendications 1 à 8,
caractérisé en ce que
l'isophoronediisocyanate est utilisé comme diisocyanate.